# EUROPEAN PATENT SPECIFICATION

(11) **EP 2 758 004 B1**
(45) Date of publication and mention of the grant of the patent: **29.07.2015**
(21) Application number: 11815861.7
(22) Date of filing: 19.09.2011
(51) Int. Cl.: A61F 2/38

(54) **TEMPORARY MODULAR SPACER DEVICE FOR JOINTS OF THE HUMAN BODY**
TEMPORÄRES MODULARES DISTANZSTÜCK FÜR GELENKE DES MENSCHLICHEN KÖRPERS
DISPOSITIF ÉCARTEUR MODULAIRE TEMPORAIRE POUR LES ARTICULATIONS DU CORPS HUMAIN

(43) Date of publication of application: 30.07.2014
(73) Proprietor: Tecres S.P.A., 37066 Sommacampagna (VR) (IT)
(72) Inventor: SOFFIATTI, Renzo, I-37066 Sommacampagna (Verona) (IT); FACCIOLI, Giovanni, I-37066 Sommacampagna (Verona) (IT)
(74) Representative: Feltrinelli, Secondo Andrea
(86) International application number: PCT/IB2011/054093
(87) International publication number: WO 2013/041905

(56) References cited:
- EP-A1- 2 347 732
- US-A1- 2004 036 189
- US-A1- 2005 085 918
- US-A1- 2005 107 885

## Description

### TECHNICAL FIELD OF THE INVENTION.

The present invention refers to a modular spacer device for the temporary replacement of articular prostheses that require to be removed for various reasons, for example due to an infection. Such modular spacer device allows, over the period of time required for treating the articulation, preserving the space required for the implantation of a new articular prosthesis and guaranteeing a good movement of the articulation.

### STATE OF THE PRIOR ART

In the field of implantology of articular prostheses it is known that such devices can be subjected to removal due to various reasons, in particular, due to local infections of the articulation after the implantation of the prosthesis.

In such case, the infected prosthesis cannot be immediately replaced with a new prosthesis, given that the seat of the articulation requires to be treated using suitable antibiotic medicines.

During the period of time required for the antibiotic treatment it is fundamental to preserve an articular space required for the implantation of a new articular prosthesis and prevent the tissues from shortening, the articulation from being subjected to atrophy and the muscles from losing tonicity.

Such technique is known as "two-stage implantation" of the articular prostheses.

There are known temporary articular spacers of the knee, manually formed by a surgeon directly during the surgical intervention of implanting the spacer. Such devices are made of the bone cement and suitably shaped, manually, in the instants preceding the implantation in the articular seat.

A drawback of such treatment method lies in the fact that the cement is formed and shaped directly during the intervention, actually increasing the duration of the operation and the difficulties for the surgeon.

Furthermore, given that in the formation of the bone cement there are used potentially toxic harmful substances, it is advisable to reduce the time of contact of the surgeon or operator therewith to the maximum.

Furthermore, the manual forming of the spacer may determine the presence of faults that may reduce the mobility offered by the articulation obtained using a similar spacer.

Pre-formed articulation spacers to be implanted without requiring any type of forming during the intervention are also available in the market. An example of such kind of pre-formed articulation spacers is disclosed in the patent application No. US 2005/085918. This application discloses a disposable articulated spacer device for treatment of joints of the human body, particularly for temporarily replacement of an expanded prosthesis, comprising one fist member (tibial member) able to be secured to a first articulation end and a second member (femoral member) able to be secured to the other articulation end. Such members are reciprocally coupled in an articulated manner to maintain a suitable joint space and at least a partial articulation for the time necessary to perform the further implantation of a joint prosthesis. However, such devices reveal the drawback of being made up of a femoral part and a tibial part combined to each other a priori, in other words they cannot be easily adapted to the anthropomorphic dimensions, even variable, of the patient. The patent application No. EP 2347732 discloses a permanent knee prosthesis system comprising a femoral component, an insert and a tibial component. The tibial component retains the insert and the insert is in turn articulated with the femoral component. These components may be provided in a plurality of sizes to accommodate at some degrees the variation of anatomies of the patient population.

A consequence of the erroneous adaptability of the known spacers to the anthropomorphic dimensions of the patient lies in the impossibility of guaranteeing a good mobility of the articulation and, thus, ensuring a good quality of life to the patient awaiting the implantation of a new articular prosthesis.

### OBJECTS OF THE INVENTION

An object of the present invention is to improve the state of the prior art.

Another object of the present invention is to provide a spacer device for the articulation of the knee that is pre-formed and which is compatible with the various dimensions or sizes of the bone ends to which it is applied.

A further object of the present invention is to provide a spacer device for the articulation of the knee that is capable of allowing a high and stable movement of the articulation in question to ensure the patient a good quality of life during the period of rehabilitation following the implantation of the spacer, even in the presence of anatomic variations between the tibial bone and the femoral bone of a patient.

According to an aspect of the present invention there is provided a modular spacer device for the articulation of the knee according to claim 1.

The dependent claims refer to preferred and advantageous embodiments of the invention.

### BRIEF DESCRIPTION OF THE DRAWINGS

Further characteristics and advantages of the present invention will be more apparent from the detailed description of a preferred but not exclusive embodiment of a modular spacer device for a knee, illustrated by way of non-limiting example in the attached drawings wherein:
figure 1 is a lateral view of the spacer device according to the present invention;
figure 2 is a perspective view of a detail of the device according to figure 1;
figure 3 is a perspective view of the detail of the device according to figure 2;
figure 4 is a perspective view of another detail of the device according to figure 1;
figure 5 is a perspective view of a further detail of the device according to figure 1;
figure 6 is a lateral view of the various sizes of the detail according to figure 2;
figure 7 is a lateral view of the various sizes of the detail according to figure 4;
figure 8 is a front view of the various sizes of the detail according to figure 5.

### EMBODIMENTS OF THE INVENTION

With reference to the attached figures, a disposable temporary modular spacer device of the articulation of a knee is schematically indicated with 1.

For the sake of further clarity, such disposable temporary modular spacer device of the articulation of a knee will be indicated hereinafter as "modular spacer device".

Such modular spacer device 1 comprises a tibial element 2, adapted to be constrained to the end of the tibial bone in proximity of the articulation of the knee, and a femoral element 4 adapted, on one side, to be constrained to the end of the femoral bone at the articulation of the knee and, on the other side, to be in contact with the tibial element 2.

The modular spacer device 1 may further comprise a shim 3. The shim 3 is optional and can be used for increasing the height of the modular spacer device 1 to compensate possible absence or strong resection of the tibial bone of the patient and/or to extend the articulation, if required.

Furthermore, the shim 3 allows offering a large surface for arranging the tibial element 2, otherwise not available, in given extreme conditions, at the sectioned end of the tibial bone of the patient.

The shim 3 is positioned at the tibial part of the patient and, in particular, beneath the tibial element 2. Thus, though obtaining the functions listed above, the shim 3 does not interfere with the articulation provided by the tibial element 2 with the femoral element 4. Thus, both in the presence of the shim 3 and when it is absent, the articulation is always preserved.

The tibial 2 and femoral 4 elements and the shim 3 are preformed and entirely made of biologically compatible material.

Such biologically compatible material is porous and it can be selected from among metals, metal alloys, organo-metallic compounds, ceramics, plastic materials and/or a combination thereof.

Specifically, the aforementioned plastic materials can be selected from among thermoplastic polymers, such as acrylic resins, polyethylene, polypropylene, polyester, etcetera, thermoformable polymers, and other similar materials.

In a version of the present invention, the biologically compatible material is a bone cement, for example of the type described in the Italian patent n° 1278853, on behalf of the applicant, incorporated herein for reference.

The aforementioned biologically compatible material, due to the porosity thereof, may be pre-impregnated, by the producer of the modular spacer device 1, using pharmaceutical and therapeutic products.

In another embodiment, the biologically compatible material, originally without medicinal substances, may be added, possibly by impregnation, using pharmaceutical and therapeutic products during the surgical intervention, in the instants preceding implantation thereof.

Still, in another embodiment, the biologically compatible material pre-impregnated by the producer of the modular spacer device 1 using pharmaceutical and therapeutic products, may be further added, during the surgical intervention, in the instants preceding implantation thereof, using pharmaceutical and therapeutic products, identical or different from those already contained therein, depending on the surgery requirements.

The shim 3 is provided with a hole 8, which is through and positioned substantially at a central position with respect to the shim 3.

The tibial element 2 is provided with a lower surface 7, substantially flat, and an upper concave surface 16.

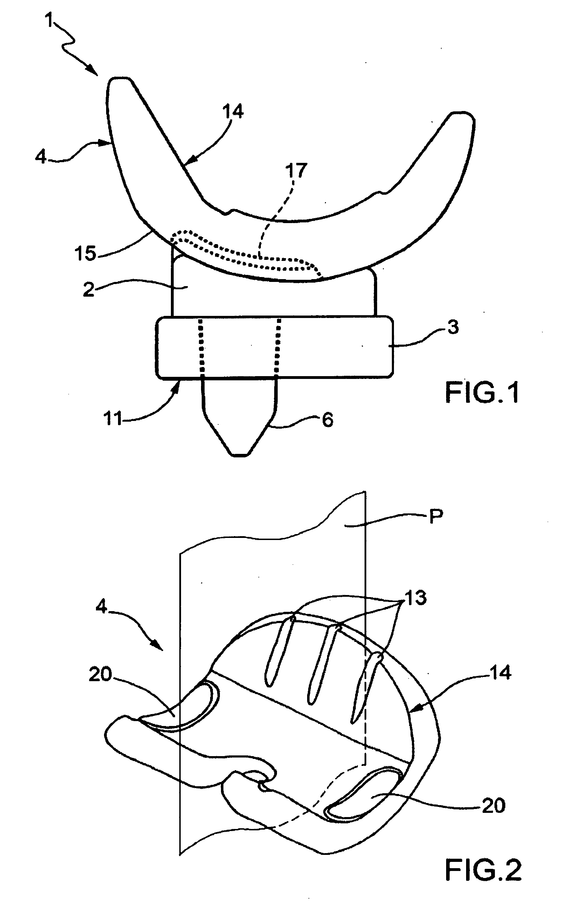

In a version of the invention, the tibial element 2, as indicated in figure 1, is provided with a substantially rod-like element 6, for example a pin or a stem, which extends, from the lower surface 7 of the tibial element 2, downwards substantially in longitudinal direction with respect to the tibial bone of the patient.

Such substantially rod-like element 6 is positioned at the hole 8 of the shim 3 and it has a diameter slightly smaller than that of the hole 8.

Thus, the substantially rod-like element 6 is adapted to be inserted into and traverse the hole 8 obtained in the shim 3.

Thus, this allows a correct relative positioning between the tibial element 2 and the shim 3, in order to guarantee coaxiality.

The tibial element 2 can be directly constrained to the portion of the tibial bone of the patient or, if present, to the shim 3. Such constraint can be obtained by means of the bone cement.

The shim 3 has an upper surface 9 and a lower surface 11. Both surfaces 9 and 11 of the shim 3 are substantially flat.

The upper surface 9 of the shim 3 is provided with first ribs 10 and respective first recesses. Such first recesses correspond to the space comprised between two adjacent first ribs 10.

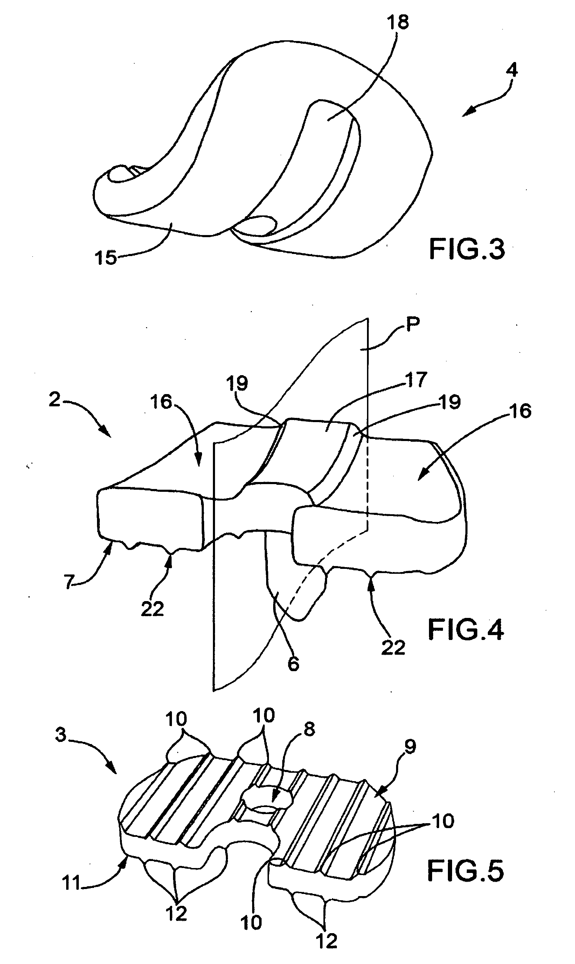

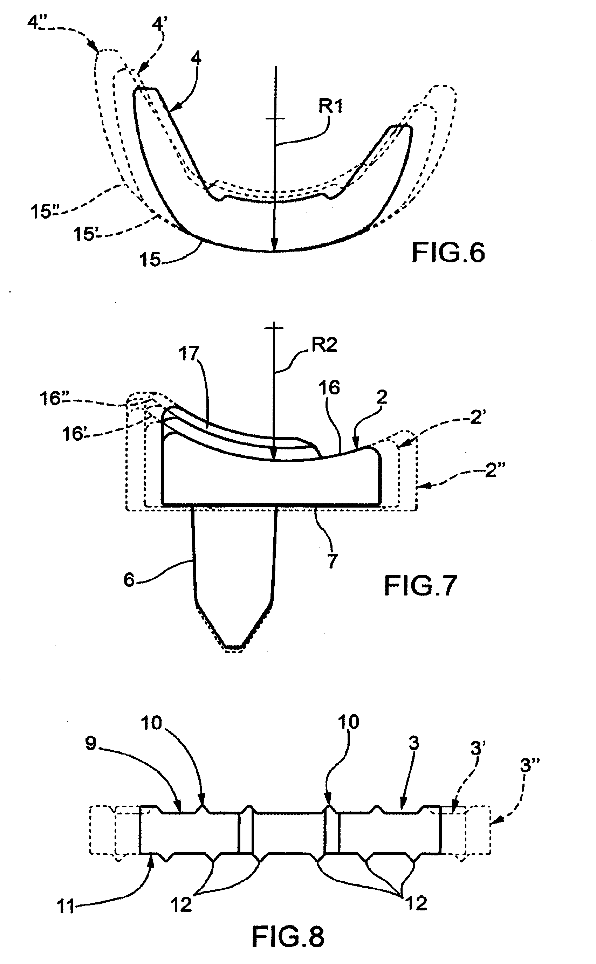

In an embodiment, as indicated in figures 5 and 8, such first ribs 10 extend linearly along the upper surface 9 of the shim 3, and they are suitably spaced from each other.

In a further version of the invention, the first ribs 10 may also develop differently, for example webbed, or having any other configuration, without departing from the scope of protection of the present invention.

The presence of such first ribs 10 and respective first recesses allows holding the bone cement, thus preventing the latter from being dispersed during the possible stage of joining the tibial element 2 with the shim 3.

The lower surface 11 of the shim 3 is provided with second ribs 12 and respective second recesses, similar to the first ribs 10 and to the first recesses described previously regarding the upper surface 9.

Such second recesses correspond to the space comprised between two adjacent second ribs 12.

The shim 3 is adapted to be constrained to the end of the tibia using bone cement.

The second ribs 12, analogously to what was described previously regarding the first ribs 10 of the upper surface 9, allow holding within the respective second recesses the bone cement and prevent the latter from entirely flowing out during the stage of implanting the shim 3 on the tibia of the patient.

The lower surface 7 of the tibial element 2 has third ribs 22 and corresponding third recesses, similar to the first ribs 10 of the upper surface 9 and to the second ribs 12 of the lower surface 11 of the shim 3.

The third recesses have the same characteristics described previously regarding the first recesses and regarding the second recesses.

In the version in which the shim 3 is not used, the surface 7 of the tibial element 2 is arranged in contact with the tibial bone of the patient. Also in this case, the third ribs 22 and the relative third recesses prevent the bone cement from entirely flowing out during the stage of implanting the tibial element 2 on the tibia of the patient.

In the version in which the shim 3 is present, the first ribs 10 of the upper surface 9 of the shim 3 are arranged at the third ribs 22 of the tibial element 2. The recesses formed thereby determine areas in which the bone cement remains, facilitating the adhesion of the tibial element 2 with the shim 3.

The first, second and third ribs, respectively 10, 12, 22, serve spacing purposes, i.e. they separate the tibial element 2 and/or the shim 3 from the tibial bone and, possibly, the tibial element 2 from the shim 3, determining an optimal minimum layer of the bone cement, under any clinical condition, so that there occurs the correct adhesion between the tibial element and/or the shim 3 and the tibial bone.

In a version of the invention, there is present at least one portion of shim 3 or more than one shim 3, depending on the specific conditions of the patient. In particular, when the tibial bone part is not parallel to the plane of the articulation, there can be inserted at least one portion of shim 3, so as to compensate the lacking bone area.

The femoral element 4 has a substantially "C-shaped" form comprising an inner concave surface 14, in contact with the bone seat, and an outer convex surface 15, adapted to enter in contact with the upper surface 16 of the tibial element 2. The femoral element 4, and in particular the inner surface 14 thereof, is adapted to be constrained to the free end of the femur using bone cement.

As indicated in the embodiment of figure 2, the femoral element 4, is provided with at least one groove 13 obtained in the inner surface 14 thereof. Such at least one groove 13 allows obtaining a better adhesion of the bone cement with the free end of the femur and with the femoral element 4.

In a particular embodiment of the femoral element 4, the inner surface 14 may comprise at least one projection 20. Such at least one projection 20 has a rounded shape and it is positioned in the lateral portions of the femoral element 4 (arranged at the lateral portions of the articulation of the knee).

Such at least one projection 20 allows obtaining an even more stable anchoring between the femoral element 4 and the femoral end itself.

Following the implantation of the modular spacer device 1, the outer surface 15 of the femoral element 4 is arranged in contact with the upper surface 16 of the tibial element 2.

The outer surface 15 of the femoral element 4 has a radius of curvature R1; the upper surface 16 of the tibial element 2 has a radius of curvature R2.

The radius of curvature R1 is smaller with respect to the radius of curvature R2.

In particular, the radius of curvature R2 is always greater than R1 and measures at least 1.5 R1.

The radii of curvature R1 and R2 are selected so as to allow combining any femoral element 4 with any tibial element 2, as better described hereinafter. In order to obtain greater stability of the articulation formed by the modular spacer device 1, the tibial element 2 comprises a projecting element 17, abutting with a hollow seat 18 obtained on the outer surface 15 of the femoral element 4.

As indicated in figure 4, the projecting element 17 extends, longitudinally to the sagittal plane of the knee, i.e. to the rotation plane P, along the upper surface 16 and it is positioned centrally with respect to the tibial element 2. Correspondingly, the seat 18 extends, longitudinally to the sagittal plane of the knee, i.e. to the rotation plane P, along the outer surface 15 and it is positioned centrally with respect to the femoral element 4. Such projecting element 17, similar to a notch, has rounded edges 19 so as to avoid damaging the femoral element 4 with which it comes to contact.

In order to facilitate the modularity of the modular spacer device 1, the projecting element 17 and the seat 18 maintain the same dimensions, regardless of whether the dimensions of the tibial element 2 and of the femoral element 4 vary, as better described hereinafter.

Should the modular spacer device 1, during movement of the articulation of the knee, be subjected to lateral thrust stress, with respect to the rotation plane P of the articulation, the projecting element 17 maintains the femoral element 4 in the seat thereof, guaranteeing a correct movement and a good stability to the articulation.

The femoral element 4, as indicated in figure 6, can be obtained in various dimensions or sizes, for example small, medium and large, as represented by the femoral element 4', 4'', reproduced using a dashed line. The femoral element 4', 4" has an outer surface 15', 15" similar to the outer surface 15 of the femoral element 4.

Though the dimensions of the femoral element 4', 4" are greater with respect to the femoral element 4, the radius of curvature R1 of the outer surface 15, is equivalent to the radius of curvature R1 of the outer surface 15', 15".

Analogously, as indicated in figures 7 and 8, there are provided further dimensions or sizes, for example small, medium and large, of the tibial element 2 and of the shim 3 represented by the tibial element 2', 2" and by the shim 3', 3" , indicated with a dashed line. The tibial element 2', 2" has an upper surface 16', 16" similar to the upper surface 16 of the tibial element 2. In particular, the radius of curvature R2 of the upper surface 16 is equivalent to the radius of curvature R2 of the upper surface 16', 16".

Substantially, varying the sizes of the femoral element 4 and the tibial element 2, the radii R1 and R2 remain constant.

The various sizes of the tibial element 2, 2', 2" and of the femoral element 4, 4', 4'' are interchangeable with respect to each other, due to the equivalence of the radius of curvature, R1 between the outer surface 15, 15', 15" and due to the equivalence of the radius of curvature R2 of the upper surface 16, 16', 16".

Purely by way of non-limiting example, a possible embodiment of the modular spacer device 1 may provide for the use of a tibial element 2 and a femoral element 4" . All possible combinations of the tibial element 2, 2', 2" and the femoral element 4, 4', 4" can be obtained though maintaining the correct stability and articulation with respect to each other.

Usually, regarding the dimensions of the shim 3, 3', 3", they correspond to those of the tibial element 2, 2', 2" used. For example, in case of use of a tibial element 2', the shim used will be 3', i.e. of the same size as the tibial element in question. Such modularity of the spacer device 1 allows adapting the latter to the anthropomorphic dimensions of the femoral and tibial ends of a patient, which may differ with respect to each other.

The characteristics indicated above regarding the femoral element 4, the tibial element 2 and the shim 3 also refer to the femoral element 4', 4" , the tibial element 2', 2" and the shim 3', 3".

The configuration of the radii of curvature R1 and R2 allows obtaining a mainly relative rolling motion between the femoral element 4, 4', 4" and the tibial element 2, 2', 2" and a partial sliding motion therebetween.

Such mainly rolling motion between the outer surface 15, 15', 15" of the femoral element 4, 4', 4" and the upper surface 16, 16', 16" of the tibial element 2, 2', 2" allows the patient to perform a flexion and extension motion of the articulation similar to the normal physiological motion of the articulation of the knee.

Adapting the modular spacer device 1 to the dimensions of each patient does not require any modification intervention of the tibial element 2, 2', 2" and of the femoral element 4, 4', 4", thus reducing the times required for the implantation of the modular spacer device 1. Actually, the surgeon is solely required to choose the best size of each element that forms the modular spacer device 1, in order to adapt each part to the actual dimensions of the respective anatomic seat of the patient, without necessarily requiring using a tibial element and a femoral element of the same size.

In order to guarantee the surgeon maximum freedom of choice, the femoral element 4, 4', 4", the tibial element 2, 2', 2" and the shim 3, 3', 3" will be packaged in separate packages and in one size.

The various configurations of the tibial element 2, 2', 2", of the shim 3, 3', 3" and of the femoral element 4, 4', 4", compatible to each other, guarantee the modular spacer device 1 a high modularity. In particular, the tibial element 2, 2', 2" is configured so as to be coupled with any size of the femoral element 4, 4', 4" to adapt the dimensions of the modular spacer device) with the dimensions of the bone ends to which it should be connected. This is obtained due to the fact that the radius R2 of the upper surface 16, 16', 16" of the tibial element 2, 2', 2" has a dimension such to be coupled rotatably and partly translatory with the radius R1, which is constant in the outer surface 15, 15', 15" of any size of the femoral element 4, 4', 4".

Therefore, the surgeon, instead of having a choice between three sizes, identical for the femoral and tibial parts, will have a wider choice, in particular nine possibilities, providing for obtaining three sizes (small, medium and large) for each femoral and tibial part separately. In addition, the possibilities increase in that it is possible to decide whether to use at least one portion of or at least one shim 3, 3', 3". Furthermore, the configuration of the aforementioned modular spacer device 1 allows obtaining a high mobility of the articulation of the knee, and a movement similar to that of a natural articulation, though in presence of different anatomic dimensions, among the various articular portions of the patient. The tibial 2 and femoral 4 elements, being preformed in various dimensions, simplify the stages of implantation thereof in the seat of the articulation, in that they do not require further forming operations or modifications of the dimensions thereof so as to be able to adapt them to the dimensions of the bone ends, actually reducing the times required for performing the surgical intervention and allowing, the patient, to have each part of the modular spacer device 1 perfectly suited to the actual bone and anatomic structure and/or compensate for possible faults due to pathologic and/or surgery conditions the patient is subjected to.

The possibility of pre-adding or adding the modular spacer device 1 using pharmacological and/or therapeutic products allows treating the local infections in the seat of the articulation and achieving the ideal conditions for implanting a new articular prosthesis.

The invention thus conceived can be subjected to numerous modifications and variants all falling within the inventive concept.

Furthermore, all details can be replaced by other technically equivalent elements. In practice, the materials used, as well as contingent shapes and dimensions may vary depending on the requirements without departing from the scope of protection of the following claims.

## Claims

1. Disposable modular spacer device (1) of the articulation of a knee comprising a tibial element (2, 2', 2"), adapted to be constrained to an end of the tibial bone in proximity of the articulation of the knee, said tibial element (2, 2', 2") comprising a lower surface (7) and an upper surface (16, 16', 16") provided with a radius of curvature R2, and a femoral element (4, 4', 4"), said femoral element (4, 4', 4") comprising an inner surface (14), adapted to be constrained to an end of the femoral bone at the articulation of the knee, and an outer convex surface (15, 15', 15"), provided with a radius of curvature R1 and adapted to enter in contact with said upper surface (16, 16', 16'') of said tibial element (2, 2', 2"), said tibial element (2, 2', 2") and femoral element (4, 4', 4") having various dimensions or sizes, **characterised in that** said tibial element (2, 2', 2") is configured so as to be coupled with any size of said femoral element (4, 4', 4") to adapt the dimensions of said modular spacer device (1) with the dimensions of the bone ends to which it should be connected.

2. Modular spacer device (1) according to claim 1, comprising a shim (3, 3', 3''), positioned beneath said tibial element (2, 2', 2").

3. Modular spacer device (1) according to claim 1 or 2, wherein said tibial element (2, 2', 2"), said femoral element (4, 4', 4") and said shim (3, 3', 3") are preformed and made of biologically compatible material.

4. Modular spacer device (1) according to the preceding claim, wherein said biologically compatible material is porous.

5. Modular spacer device (1) according to any one of the preceding claims, wherein said biologically compatible material comprises at least one among the following materials: metals, metal alloys, organo-metallic compounds, ceramics, plastic materials, such as thermoplastic polymers, acrylic resins, polyethylene, polypropylene, polyesters, etcetera, thermoformable polymers, other similar materials, bone cement and/or a combination thereof.

6. Modular spacer device (1) according to any one of the preceding claims, wherein said upper surface (16, 16', 16") is concave and said lower surface (7) is substantially flat.

7. Modular spacer device (1) according to any one of the preceding claims, wherein said radius of curvature R1 is smaller with respect to said radius of curvature R2.

8. Modular spacer device (1) according to any one of the preceding claims, wherein said radius of curvature R2 measures at least 1.5 R1.

9. Modular spacer device (1) according to any one of the preceding claims, wherein said shim (3, 3', 3") has an upper surface (9) and a lower surface (11), substantially flat, and/or a hole (8).

10. Modular spacer device (1) according to any one of the preceding claims, wherein said tibial element (2, 2', 2") comprises a substantially rod-like element (6) which is extended from said lower surface (7) downwards substantially in longitudinal direction with respect to the tibial bone of the patient and which is shaped similar to a pin or a stem or to a similar element, configured so as to be inserted into said hole (8).

11. Modular spacer device (1) according to any one of the preceding claims, wherein said tibial element (2, 2', 2") is adapted to be constrained to the tibial bone of the patient using bone cement.

12. Modular spacer device (1) according to any one of claims 1-10, wherein said tibial element (2, 2', 2") and said shim (3, 3', 3") are adapted to be constrained to each other using bone cement and said shim (3, 3', 3") is adapted to be constrained to the tibial bone of the patient using bone cement.

13. Modular spacer device (1) according to any one of the preceding claims, wherein said shim (3, 3', 3") has, in said upper surface (9), first ribs (10) and relative first recesses and/or wherein said tibial element (2, 2', 2") has, in said lower surface (7), third ribs (22) and corresponding third recesses, for holding said bone cement.

14. Modular spacer device (1) according to any one of the preceding claims, wherein said shim (3, 3', 3") has, in said lower surface (11), second ribs (12) and relative second recesses for the retention of said bone cement and for constraining said shim (3, 3', 3") with the tibial bone of the patient.

15. Modular spacer device (1) according to any one of the preceding claims, wherein said inner surface (14) of said femoral element (4, 4', 4") is provided with at least one groove (13) and/or of at least one projection (20) for a better adhesion of said inner surface (14) with the femoral bone of a patient, possibly by using bone cement.

16. Modular spacer device (1) according to any one of the preceding claims, wherein said outer surface (15, 15', 15"), of the radius R1, is in rotary and partly translatory contact with said upper concave surface (16, 16', 16") of the radius R2.

17. Modular spacer device (1) according to any one of the preceding claims, wherein said outer surface (15, 15', 15") comprises a hollow seat (18).

18. Modular spacer device (1) according to any one of the preceding claims, wherein said tibial element (2, 2', 2") comprises a projecting element (17), abutting with said seat (18), for stabilising the movement of said modular spacer device (1).

19. Modular spacer device (1) according to any one of the preceding claims, wherein said projecting element (17) extends, longitudinally to the sagittal plane of the knee, along said upper surface (16, 16', 16") of said tibial element (2, 2', 2").

20. Modular spacer device (1) according to any one of the preceding claims, wherein said projecting element (17) has rounded edges (19).

21. Modular spacer device (1) according to claim 4, wherein said porous biologically compatible material is adapted to be pre-impregnated, by the producer of the modular spacer device 1, using pharmaceutical and therapeutic products.

22. Modular spacer device (1) according to claim 4, wherein said porous biologically compatible material, originally without medicinal substances, is adapted to be impregnated using pharmaceutical and therapeutic products before the implantation of said device.

23. Modular spacer device (1) according to claim 21, wherein said porous biologically compatible material is adapted to be impregnated using pharmaceutical and therapeutic products before the implant of said device.

## Patentansprüche

1. Austauschbares modulares Distanzstück (1) für das Gelenk eines Knies, das ein Tibiaelement (2, 2', 2"), das an einem Ende des tibialen Knochens in der Nähe des Gelenks des Knies befestigt werden kann, wobei dieses Tibiaelement (2, 2', 2") eine untere Oberfläche (7) und eine mit einem Krümmungsradius R2 versehene obere Oberfläche (16, 16', 16") umfasst, und ein Femurelement (4, 4', 4") umfasst, wobei dieses Femurelement (4, 4', 4") eine innere Oberfläche (14), die an einem Ende des Femurknochens beim Gelenk des Knies befestigt werden kann, und eine äußere konvexe Oberfläche (15, 15', 15") umfasst, die mit einem Krümmungsradius R1 versehen und geeignet ist, in Kontakt mit der oberen Oberfläche (16, 16', 16") des Tibiaelements (2, 2', 2") zu kommen, wobei das Tibiaelement (2, 2', 2") und das Femurelement (4, 4', 4") unterschiedliche Abmessungen oder Größen haben, **dadurch gekennzeichnet, dass** das Tibiaelement (2, 2', 2") gestaltet ist, um mit einer beliebigen Größe des Femurelements (4, 4', 4") verbunden zu werden, um die Abmessungen des modularen Distanzstücks (1) an die Abmessungen der Knochenenden anzupassen, mit denen es verbunden werden soll.

2. Modulares Distanzstück (1) nach Anspruch 1, das eine Zwischenscheibe (3, 3', 3") umfasst, die unter dem Tibiaelement (2, 2', 2") angeordnet ist.

3. Modulares Distanzstück (1) nach Anspruch 1 oder 2, wobei das Tibiaelement (2, 2', 2"), das Femurelement (4, 4', 4") und die Zwischenscheibe (3, 3', 3") vorgeformt sind und aus einem biologisch verträglichen Material bestehen.

4. Modulares Distanzstück (1) nach dem vorhergehenden Anspruch, wobei das biologisch verträgliche Material porös ist.

5. Modulares Distanzstück (1) nach einem der vorhergehenden Ansprüche, wobei das biologisch verträgliche Material mindestens eines der folgenden Materialien umfasst: Metalle, Metalllegierungen, metallorganische Verbindungen, Keramik, Kunststoffe wie thermoplastische Polymere, Acrylharze, Polyethylen, Polypropylen, Polyester usw., thermoplastisch verformbare Polymere, andere gleichartige Materialien, Knochenzement und/oder eine Kombination davon.

6. Modulares Distanzstück (1) nach einem der vorhergehenden Ansprüche, wobei die obere Oberfläche (16, 16', 16") konkav ist und die untere Oberfläche (7) im Wesentlichen plan ist.

7. Modulares Distanzstück (1) nach einem der vorhergehenden Ansprüche, wobei der Krümmungsradius R1 kleiner als der Krümmungsradius R2 ist.

8. Modulares Distanzstück (1) nach einem der vorhergehenden Ansprüche, wobei der Krümmungsradius R2 mindestens das 1,5-Fache von R1 beträgt.

9. Modulares Distanzstück (1) nach einem der vorhergehenden Ansprüche, wobei die Zwischenscheibe (3, 3', 3") eine obere Oberfläche (9) und eine untere Oberfläche (11), die im Wesentlichen plan sind, und/oder ein Loch (8) aufweist.

10. Modulares Distanzstück (1) nach einem der vorhergehenden Ansprüche, wobei das Tibiaelement (2, 2', 2") ein im Wesentlichen stabähnliches Element (6) umfasst, das sich von der unteren Oberfläche (7) im Wesentlichen in Längsrichtung zum tibialen Knochen des Patienten nach unten erstreckt und das ähnlich wie ein Zapfen oder eine Stange oder ein gleichartiges Element geformt ist, das gestaltet ist, um in das Loch (8) eingeführt zu werden.

11. Modulares Distanzstück (1) nach einem der vorhergehenden Ansprüche, wobei das Tibiaelement (2, 2', 2") geeignet ist, mit Knochenzement am tibialen Knochen des Patienten befestigt zu werden.

12. Modulares Distanzstück (1) nach einem der Ansprüche von 1 bis 10, wobei das Tibiaelement (2, 2', 2") und die Zwischenscheibe (3, 3', 3") geeignet sind, mit Knochenzement aneinander befestigt zu werden, und die Zwischenscheibe (3, 3', 3") geeignet ist, mit Knochenzement am tibialen Knochen des Patienten befestigt zu werden.

13. Modulares Distanzstück (1) nach einem der vorhergehenden Ansprüche, wobei die Zwischenscheibe (3, 3', 3") auf der oberen Oberfläche (9) erste Rippen (10) und entsprechende erste Vertiefungen aufweist, und/oder wobei das Tibiaelement (2, 2', 2") auf der unteren Oberfläche (7) dritte Rippen (22) und entsprechende dritte Vertiefungen zum Halten des Knochenzements aufweist.

14. Modulares Distanzstück (1) nach einem der vorhergehenden Ansprüche, wobei die Zwischenscheibe (3, 3', 3") auf der unteren Oberfläche (11) zweite Rippen (12) und entsprechende zweite Vertiefungen zum Zurückhalten des Knochenzements und zum Befestigen der Zwischenscheibe (3, 3', 3") am tibialen Knochen des Patienten aufweist.

15. Modulares Distanzstück (1) nach einem der vorhergehenden Ansprüche, wobei die innere Oberfläche (14) des Femurelements (4, 4', 4") mit mindestens einer Nut (13) und/oder mindestens einem Vorsprung (20) für eine bessere Haftung der inneren Oberfläche (14) am Femurknochen eines Patienten, möglichst unter Verwendung von Knochenzement, versehen ist.

16. Modulares Distanzstück (1) nach einem der vorhergehenden Ansprüche, wobei sich die äußere Oberfläche (15, 15', 15") mit dem Radius R1 in einem rotatorischen und teilweise translatorischen Kontakt mit der oberen konkaven Oberfläche (16, 16', 16") mit dem Radius R2 befindet.

17. Modulares Distanzstück (1) nach einem der vorhergehenden Ansprüche, wobei die äußere Oberfläche (15, 15', 15") eine hohle Aufnahme (18) umfasst.

18. Modulares Distanzstück (1) nach einem der vorhergehenden Ansprüche, wobei das Tibiaelement (2, 2', 2") ein vorstehendes Element (17) umfasst, das an der Aufnahme (18) anliegt, um die Bewegung des modularen Distanzstücks (1) zu stabilisieren.

19. Modulares Distanzstück (1) nach einem der vorhergehenden Ansprüche, wobei sich das vorstehende Element (17) in Längsrichtung zur Sagittalebene des Knies entlang der oberen Oberfläche (16, 16', 16") des Tibiaelements (2, 2', 2") erstreckt.

20. Modulares Distanzstück (1) nach einem der vorhergehenden Ansprüche, wobei das vorstehende Element (17) abgerundete Kanten (19) aufweist.

21. Modulares Distanzstück (1) nach Anspruch 4, wobei das poröse biologisch verträgliche Material geeignet ist, vom Hersteller des modularen Distanzstücks (1) mit pharmazeutischen und therapeutischen Produkten vorimprägniert zu werden.

22. Modulares Distanzstück (1) nach Anspruch 4, wobei das poröse biologisch verträgliche Material, das ursprünglich keine medizinischen Substanzen aufweist, geeignet ist, vor der Implantation der Vorrichtung mit pharmazeutischen und therapeutischen Produkten imprägniert zu werden.

23. Modulares Distanzstück (1) nach Anspruch 21, wobei das poröse biologisch verträgliche Material geeignet ist, vor der Implantation der Vorrichtung mit pharmazeutischen und therapeutischen Produkten imprägniert zu werden.

## Revendications

1. Dispositif écarteur modulaire jetable (1) de l'articulation d'un genou comprenant un élément tibial (2, 2', 2"), adapté pour être fixé à une extrémité du tibia à proximité de l'articulation du genou, ledit élément tibial (2, 2', 2") comprenant une surface inférieure (7) et une surface supérieure (16, 16', 16") dotée d'un rayon de courbure R2, et un élément fémoral (4, 4', 4"), ledit élément fémoral (4, 4', 4") comprenant une surface intérieure (14), adaptée pour être fixée à une extrémité du fémur à l'articulation du genou, et une surface extérieure convexe (15, 15', 15") dotée d'un rayon de courbure R1 et adaptée pour venir en contact avec ladite surface supérieure (16, 16', 16") dudit élément tibial (2, 2', 2"), ledit élément tibial (2, 2', 2") et ledit élément fémoral (4, 4', 4") ayant diverses dimensions ou tailles, **caractérisé en ce que** ledit élément tibial (2, 2', 2") est configuré de manière à être couplé avec n'importe quelle taille dudit élément fémoral (4, 4', 4") pour adapter les dimensions dudit dispositif écarteur modulaire (1) aux dimensions des extrémités osseuses auxquelles il doit être connecté.

2. Dispositif écarteur modulaire (1) selon la revendication 1, comprenant une cale (3, 3', 3 "), positionnée au-dessous dudit élément tibial (2, 2', 2").

3. Dispositif écarteur modulaire (1) selon la revendication 1 ou 2, dans lequel ledit élément tibial (2, 2', 2"), ledit élément fémoral (4, 4', 4") et ladite cale (3, 3', 3") sont préformés et constitués de matériau biologiquement compatible.

4. Dispositif écarteur modulaire (1) selon la revendication précédente, dans lequel ledit matériau biologiquement compatible est poreux.

5. Dispositif écarteur modulaire (1) selon l'une quelconque des revendications précédentes, dans lequel ledit matériau biologiquement compatible comprend au moins un parmi les matériaux suivants : métaux, alliages métalliques, composés organométalliques, céramiques, matières plastiques, telles que des polymères thermoplastiques, résines acryliques, polyéthylène, polypropylène, polyesters, etcetera, polymères thermoformables, autres matériaux similaires, ciment osseux et/ou une combinaison de ceux-ci.

6. Dispositif écarteur modulaire (1) selon l'une quelconque des revendications précédentes, dans lequel ladite surface supérieure (16, 16', 16") est concave et ladite surface inférieure (7) est sensiblement plate.

7. Dispositif écarteur modulaire (1) selon l'une quelconque des revendications précédentes, dans lequel ledit rayon de courbure R1 est plus petit par rapport audit rayon de courbure R2.

8. Dispositif écarteur modulaire (1) selon l'une quelconque des revendications précédentes, dans lequel ledit rayon de courbure R2 mesure au moins 1,5 R1.

9. Dispositif écarteur modulaire (1) selon l'une quelconque des revendications précédentes, dans lequel ladite cale (3, 3', 3") comporte une surface supérieure (9) et une surface inférieure (11), sensiblement plate, et/ou un trou (8).

10. Dispositif écarteur modulaire (1) selon l'une quelconque des revendications précédentes, dans lequel ledit élément tibial (2, 2', 2") comprend un élément sensiblement en forme de tige (6) qui s'étend à partir de ladite surface inférieure (7) vers le bas sensiblement en direction longitudinale par rapport au tibia du patient et qui est profilé de manière similaire à une goupille ou une tige ou à un élément similaire, configuré de manière à être inséré dans ledit trou (8).

11. Dispositif écarteur modulaire (1) selon l'une quelconque des revendications précédentes, dans lequel ledit élément tibial (2, 2', 2") est adapté pour être fixé au tibia du patient en utilisant du ciment osseux.

12. Dispositif écarteur modulaire (1) selon l'une quelconque des revendications 1 à 10, dans lequel ledit élément tibial (2, 2', 2") et ladite cale (3, 3', 3 ") sont adaptés pour être fixés ensemble en utilisant du ciment osseux et ladite cale (3, 3', 3") est adaptée pour être fixée au tibia du patient en utilisant du ciment osseux.

13. Dispositif écarteur modulaire (1) selon l'une quelconque des revendications précédentes, dans lequel ladite cale (3, 3', 3") comporte, dans ladite surface supérieure (9), des premières nervures (10) et des premiers évidements correspondants et/ou dans lequel ledit élément tibial (2, 2', 2") comporte, dans ladite surface inférieure (7), des troisièmes nervures (22) et des troisièmes évidements correspondants, pour maintenir ledit ciment osseux.

14. Dispositif écarteur modulaire (1) selon l'une quelconque des revendications précédentes, dans lequel ladite cale (3, 3', 3") comporte, dans ladite surface inférieure (11), des deuxièmes nervures (12) et des deuxièmes évidements correspondants pour la rétention dudit ciment osseux et pour bloquer ladite cale (3, 3', 3") avec le tibia du patient.

15. Dispositif écarteur modulaire (1) selon l'une quelconque des revendications précédentes, dans lequel ladite surface intérieure (14) dudit élément fémoral (4, 4', 4") est pourvue d'au moins une rainure (13) et/ou d'au moins une saillie (20) pour une meilleure adhérence de ladite surface intérieure (14) avec le fémur d'un patient, éventuellement en utilisant du ciment osseux.

16. Dispositif écarteur modulaire (1) selon l'une quelconque des revendications précédentes, dans lequel ladite surface extérieure (15, 15', 15"), du rayon R1, est en contact en rotation et partiellement en translation avec ladite concave surface supérieure (16, 16', 16") du rayon R2.

17. Dispositif écarteur modulaire (1) selon l'une quelconque des revendications précédentes, dans lequel ladite surface extérieure (15, 15', 15") comprend un siège creux (18).

18. Dispositif écarteur modulaire (1) selon l'une quelconque des revendications précédentes, dans lequel ledit élément tibial (2, 2', 2") comprend un élément en saillie (17), en butée avec ledit siège (18), pour stabiliser le mouvement dudit dispositif écarteur modulaire (1).

19. Dispositif écarteur modulaire (1) selon l'une quelconque des revendications précédentes, dans lequel ledit élément en saillie (17) s'étend, longitudinalement par rapport au plan sagittal du genou, le long de ladite surface supérieure (16, 16', 16") dudit élément tibial (2, 2', 2").

20. Dispositif écarteur modulaire (1) selon l'une quelconque des revendications précédentes, dans lequel ledit élément en saillie (17) a des bords arrondis (19).

21. Dispositif écarteur modulaire (1) selon la revendication 4, dans lequel ledit matériau biologiquement compatible poreux est adapté pour être pré-imprégnée, par le producteur du dispositif écarteur modulaire (1), en utilisant des produits pharmaceutiques et thérapeutiques.

22. Dispositif écarteur modulaire (1) selon la revendication 4, dans lequel ledit matériau biologiquement compatible poreux, initialement sans substances médicamenteuses, est adapté pour être imprégnée en utilisant des produits pharmaceutiques et thérapeutiques avant l'implantation dudit dispositif.

23. Dispositif écarteur modulaire (1) selon la revendication 21, dans lequel ledit matériau biologiquement compatible poreux est adapté pour être imprégnée en utilisant des produits pharmaceutiques et thérapeutiques avant l'implantation dudit dispositif.
